Europäisches Patentamt

European Patent Office

Office européen des brevets

(19)

(11) Numéro de publication: **0 358 588**
**A1**

(12) **DEMANDE DE BREVET EUROPEEN**

(21) Numéro de dépôt: 89470016.0

(22) Date de dépôt: 25.08.89

(51) Int. Cl.⁵: **A 61 M 16/01**

(30) Priorité: 31.08.88 FR 8811566

(43) Date de publication de la demande:
14.03.90 Bulletin 90/11

(84) Etats contractants désignés:
AT BE CH DE ES GB IT LI NL SE

(71) Demandeur: **Vivant, Jean-Francois**
**7 rue Majorelle**
**F-54000 Nancy (FR)**

(72) Inventeur: **Vivant, Jean-Francois**
**7 rue Majorelle**
**F-54000 Nancy (FR)**

(74) Mandataire: **Poupon, Michel**
**3, rue Thiers BP 421**
**F-88011 Epinal Cédex (FR)**

(54) Dispositif pour l'aspiration et l'évacuation des gaz anesthésiques.

(57) Dispositif pour l'aspiration et l'évacuation des gaz anesthésiques, tels que protoxyde d'azote et organohalogénés, exhalés par un patient au cours d'une intervention sous anesthésie, qu'elle soit en ventilation assistée, contrôlée ou spontanée, ou qu'elle fasse intervenir un respirateur. Il consiste en un tube en serpentin (7) à une extrémité duquel sont amenés les gaz exhalés, directement ou indirectement, par le patient et à l'intérieur duquel est réalisée une aspiration à une certaine distance de l'endroit d'arrivée des gaz dans ledit serpentin, par un tube d'aspiration (2) dans lequel règne une légère dépression de l'ordre de 650 millibars, ledit tube d'aspiration aspirant ainsi les gaz anesthésiques qui sont évacués par le compresseur dans la centrale de vide.

FIG. 2

EP 0 358 588 A1

**Description**

## DISPOSITIF POUR L'ASPIRATION ET L'EVACUATION DES GAZ ANESTHESIQUES

La présente invention a pour objet un dispositif pour l'aspiration et l'évacuation des gaz anesthésiques, tels que protoxyde d'azote et organohalogénés, exhalés par un patient au cours· d'une intervention sous anesthésie, quelle soit en ventilation assistée contrôlée ou spontanée, ou qu'elle fasse intervenir un respirateur.

Lors d'une intervention sous anesthésie générale, le patient est souvent amené à respirer un certain nombre de produits gazeux, en particulier du protoxyde d'azote et des hydrocarbures halogénés. Ces techniques sont en elles-mêmes bien connues de l'homme de l'art et ne seront donc pas décrites plus en détail. Les gaz servent à anesthésier et ventiler le patient, et sont ensuite rejetés par celui-ci, directement ou indirectement selon le mode opératoire anesthésique mis en oeuvre, dans l'atmosphère du bloc opératoire ou des salles de réveil et d'induction.

Il en découle pour le personnel présent quotidiennement dans ces lieux des nuisances certaines, même si les concentrations en ces produits restent faibles, du fait de la durée quotidienne d'exposition et de la répétition de cette exposition tout au long de l'année. Des troubles parfois graves peuvent apparaître encore peu ou mal connus, tels que fatigabilité, maux de tête, troubles hépatiques, états dépressifs, insomnie, teratotoxicité, etc...

Le problème se pose donc de l'évacuation des gaz anesthésiques exhalés par le patient.

Un certain nombre de systèmes ont déjà été proposés, mais aucun ne donne entière satisfaction, soit parce qu'ils sont réellement trop onéreux et complexes (donc sources de mauvais fonctionnements), soit parce qu'ils ne peuvent s'adapter à tous les matériels nouveaux ou existants, ni à tous les types d'anesthésies.

La présente invention se propose de remédier à ces inconvénients des dispositifs de l'art antérieur.

Conformément à l'invention, ce résultat est obtenu avec un dispositif pour l'aspiration et l'évacuation des gaz anesthésiques, tels que protoxyde d'azote et organohalogénés, exhalés par un patient au cours d'une intervention sous anesthésie, qu'elle soit en ventilation assistée contrôlée ou spontanée, ou qu'elle fasse intervenir un respirateur, caractérisé en ce qu'il consiste en un tube en serpentin à une extrémité duquel sont amenés les gaz exhalés, directement ou indirectement, par le patient et à l'intérieur duquel est réalisée une aspiration à une certaine distance de l'endroit d'arrivée des gaz dans ledit serpentin, par un tube d'aspiration dans lequel règne une légère dépression de l'ordre de 650 millibars, ledit tube d'aspiration aspirant ainsi les gaz anesthésiques qui sont évacués par le compresseur dans la centrale de vide.

Selon un mode de mise en oeuvre, le tube d'aspiration sera extérieur au tube en serpentin et sera relié à celui-ci par un piquage.

Selon une autre variante de mise en oeuvre, le tube d'aspiration sera disposé à l'intérieur dudit tube en serpentin.

Grâce au dispositif selon l'invention, on réalise une réelle aspiration continue et l'évacuation des gaz anesthésiques dans toutes les conditions de mise en oeuvre de l'anesthésie :
- soit à la sortie expiratoire d'un respirateur fonctionnant en circuit ouvert ;
- soit à la sortie d'une valve d'anesthésie type "AMBU" lorsque l'on effectue une anesthésie gazeuse en circuit ouvert en ventilation manuelle contrôlée, assistée ou en ventilation spontanée.

L'évacuation des gaz aspirés se fait par une aspiration murale reliée à la centrale de vide du bloc opératoire (pression négative de moins 650 millibars) ou par un venturi (air comprimé du bloc).

Le tube en serpentin sera par exemple un tube annelé en PVC transparent, et le tube d'aspiration sera un tube souple transparent en PVC. Le tube sera conformé en serpentin (ce qui est aisé en raison des anneaux) et maintenu par des thermocolles classiques.

Ce système présente de multiples avantages :
- il diminue notablement la pollution de l'atmosphère du bloc opératoire en évacuant de façon continue les gaz anesthésiques toxiques (protoxyde d'azote et halogénés). Il assure donc le confort des personnels travaillant dans le bloc opératoire (absence de céphalées et d'hépatotoxicité par les halogénés, absence d'effet tératogène du protoxyde d'azote) ;
- absence de réglage ;
- l'aspiration des gaz toxiques est continue et constante ;
- coût de fabrication quasiment nul (technologie très simple) ;
- entretien nul. Système pouvant être éventuellement désinfecté à froid au formol ou à l'autoclave si l'on adopte un montage amovible et une tuyauterie en plastique compatible avec ce mode de stérilisation ;
- encombrement réduit. Ce faible encombrement permet une intégration du système dans la carrosserie protégeant les respirateurs modernes ;
- il s'adapte sur tous les types de respirateurs fonctionnant en circuit ouvert ;
- sur les respirateurs anciens, il peut être accolé à l'appareil sous forme d'un module distinct ;
- si le système n'est pas mis en aspiration, il ne gêne en rien l'évacuation des gaz par le respirateur ;
- le système est utilisable en ventilation manuelle contrôlée ou assistée et en ventilation spontanée. Il est alors nécessaire de le relier à la valve d'anesthésie par un tuyau adapté. Le malade peut être ventilé au masque ou être intubé ;
- absence de retentissement sur le respirateur ou la valve d'AMBU :
* pression négative extrêmement faible (inférieure ou égale à moins 0,2 cm d'eau en dehors du temps expiratoire de l'appareil),
* impossibilité de surpression dans le système, car il n'y a pas de valve de surpression donc absence de risque de blocage de ce genre de valve (pression inférieure ou égale à plus 1 cm d'eau au temps expiratoire de l'appareil).

- le rendement du système est excellent, ce qui est vérifié par les mesures des taux en protoxyde d'azote et en halogénés au début et en fin de circuit ;

- le système remplace avantageusement les filtres à charbon actif utilisés pour fixer les halogénés (dispositifs onéreux, peu pratiques, relargant ces produits toxiques au bout d'un certain temps d'utilisation). D'autre part, ces filtres à charbon actif ne fixent pas le protoxyde d'azote.

Il pourra être utilisé tant en position horizontale que verticale. On préférera la position verticale lorsque risquent de se produire des phénomènes de condensation. L'appareil sera alors muni d'un piège à eau de type en lui-même connu.

On comprendra mieux l'invention à l'aide de la description faite ci-après d'un certain nombre de caractéristiques de mise en oeuvre du dispositif conforme à l'invention, données à titre d'exemples non limitatifs, en référence aux dessins annexés dans lesquels :

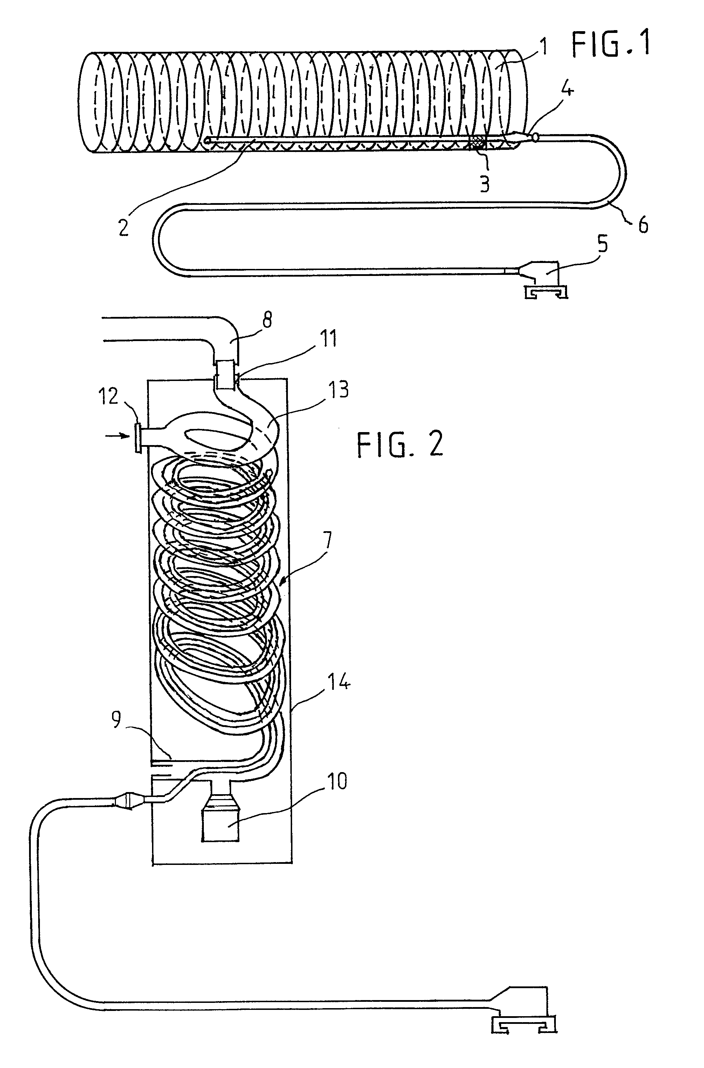

- la figure 1 illustre un mode de réalisation du tube en serpentin et du tube d'aspiration ;

- la figure 2 est une vue schématique d'un dispositif conforme à l'invention, utilisé en position verticale.

Le dispositif d'aspiration conforme à l'invention est obtenu à partir d'un tube annelé (1), les anneaux étant conformés sur la partie externe du tube, sa paroi interne étant lisse.

A l'intérieur de ce premier tube est fixé un tube plus fin (2) d'aspiration, fixé par exemple par points de colle (3), raccordé par un raccord conique (4) à une prise murale de vide (5) par un tuyau (6) renforcé, indéformable, antistatique. A titre d'exemple, le tube (1) pourra avoir un diamètre de 22 mm et le tube (2) un diamètre des 5 mm.

Les deux tubes pourront être en tout matériau compatible avec l'utilisation envisagée, par exemple en PVC transparent.

Grâce à sa structure annelée, le tube (1) peut être conformé en serpentin (7) tel que représenté à la figure 2. Les spires du serpentin seront de préférence jointives et solidarisées par une colle adéquate.

le tube (1) en serpentin (7) est relié à l'une de ses extrémités au tuyau de sortie (8) d'un respirateur, non représenté. L'autre extrémité (9) est à l'air libre.

En partie basse, le tube (1) comporte un piège à eau (10) qui permet d'évacuer facilement l'eau de condensation éventuelle qui descend par gravité dans le serpentin (7).

Le tube d'aspiration (2) débouchera en partie haute du serpentin à une certaine distance du tuyau de sortie du respirateur, de manière à limiter au maximum les perturbations pouvant être générées en amont sur le respirateur, ménageant ainsi une zone de réserve (13).

En partie haute, au niveau de son extrémité de raccordement (11) au respirateur, le serpentin sera muni d'un orifice (12) pouvant être à volonté fermé ou ouvert et permettant de relier par un tuyau adapté la valve d'AMBU au système dans le cas de l'utilisation d'une ventilation spontanée ou assistée (malade intubé ou ventilé au masque ).

Le tube d'aspiration (2) pourra également être collé sur une partie de son parcours à l'extérieur du serpentin et ne pénétrer dans celui-ci qu'en amont de la zone de réserve (13) par rapport au tuyau de sortie du respirateur.

L'ensemble pourra être disposé dans un carter transparent (14).

**Revendications**

1. Dispositif pour l'aspiration et l'évacuation des gaz anesthésiques, tels que protoxyde d'azote et organohalogénés, exhalés par un patient au cours d'une intervention sous anesthésie, qu'elle soit en ventilation assistée, contrôlée ou spontanée, ou qu'elle fasse intervenir un respirateur, caractérisé en ce qu'il consiste en un tube (1) en serpentin (7) à une extrémité duquel sont amenés les gaz exhalés, directement ou indirectement, par le patient et à l'intérieur duquel est réalisée une aspiration à une certaine distance de l'endroit d'arrivée des gaz dans ledit serpentin, par un tube d'aspiration (2) dans lequel règne une légère dépression de l'ordre de 650 millibars, ledit tube d'aspiration aspirant ainsi les gaz anesthésiques qui sont évacués par le compresseur dans la centrale de vide.

2. Dispositif selon la revendication 1, caractérisé en ce que le tube d'aspiration est extérieur au serpentin (7) et qu'il est relié à celui-ci par un piquage.

3. Dispositif selon la revendication 1, caractérisé en ce que le tube d'aspiration est disposé à l'intérieur du serpentin (7).

4. Dispositif selon l'une quelconque des revendications 1 à 3, caractérisé en ce que le tube (1) est un tube annelé.

5. Dispositif selon l'une quelconque des revendications I à 4, caractérisé en ce qu'il est disposé verticalement.

6. Dispositif selon la revendication 5, caractérisé en ce qu'il comporte un piège à eau (10).

7. Dispositif selon l'une quelconque des revendications 1 à 6, caractérisé en ce qu'il comporte, au niveau de l'extrémité de raccordement (11) au respirateur, un orifice (12) pour l'adaptation à une valve pour ventilation spontanée ou assistée.

FIG.1

FIG. 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 266 529 (MÖLLSTAM)<br>* Colonne 1, ligne 23 - colonne 2, ligne 4; colonne 2, lignes 39-44; colonne 4, lignes 22-43; figures 1,4 *<br>--- | 1-5 | A 61 M 16/01 |
| A | US-A-4 527 558 (HOENIG)<br>* Colonne 2, lignes 25-68; colonne 3, ligne 31 - colonne 4, ligne 45; figures 2,3 *<br>--- | 1,6 | |
| A | GB-A-2 174 607 (AUTOMATED PROCESS AND CONTROL)<br>* Page 1, lignes 23-33; page 1, lignes 56-88; figures 1,2 *<br>--- | 1,3,4 | |
| A | US-A-4 320 754 (WATSON et al.)<br>* Colonne 2, lignes 18-25; colonne 2, lignes 48-58; colonne 4, lignes 34-39; figures 2,6,6a *<br>----- | 1 | |

DOMAINES TECHNIQUES
RECHERCHES (Int. Cl.5)

A 61 M

Le présent rapport a été établi pour toutes les revendications

| Lieu de la recherche | Date d'achèvement de la recherche | Examinateur |
|---|---|---|
| LA HAYE | 06-12-1989 | SCHOENLEBEN J.E.F. |

EPO FORM 1503 03.82 (P0402)